Europäisches Patentamt

(19) European Patent Office

Office européen des brevets

(11) Publication number: **0 268 465**
**A2**

# (12) EUROPEAN PATENT APPLICATION

(21) Application number: **87310172.9**

(22) Date of filing: **18.11.87**

(51) Int. Cl.⁴: **G 01 N 33/53**
**G 01 N 33/543**

(30) Priority: **19.11.86 ZA 868772**

(43) Date of publication of application:
**25.05.88 Bulletin 88/21**

(84) Designated Contracting States:
**AT CH DE ES GR IT LI**

(71) Applicant: **BIOCLONES (PROPRIETARY) LIMITED**
**142 Medical Mews**
**Sandton City Sandton Transvaal (ZA)**

(72) Inventor: **Rowland, George Ferdinand**
**2 Shiraz Street**
**Stellenbosch 7600 Cape Province (ZA)**

**Engelbrecht, David Josephus**
**Nippon Jonkershoek Road**
**Stellenbosch Cape Province (ZA)**

(74) Representative: **Holmes, Michael John et al**
**Frank B. Dehn & Co. European Patent Attorneys Imperial**
**House 15-19 Kingsway**
**London, WC2B 6UZ, (GB)**

(54) Method for detecting the presence of a plant antigen in a plant.

(57) A method of detecting the presence of a plant antigen in or on a plant, e.g. the detection of Grapevine Virus A, includes immobilising a coating antibody to GVA on a surface, bringing an extract of the grapevine into contact with the coating antibody to allow the GVA which may be present in the grapevine extract to be bound by the coating antibody, bringing a detecting antibody to GVA into contact with the bound GVA to allow the GVA to be bound to the detecting antibody, bringing a bridging antibody into contact with the detecting antibody to allow the bridging antibody to be bound to the detecting antibody, bringing an enzyme anti-enzyme antibody complex (e.g. PAP) into contact with the detecting antibody to allow the enzyme anti-enzyme antibody complex to be bound to the bridging antibody, bringing a substrate for the enzyme into contact with the bound enzyme anti-enzyme antibody complex, and detecting any change of the substrate. This method provides increased sensitivity, reproducibility and simplicity.

EP 0 268 465 A2

## Description

## "METHOD OF DETECTING THE PRESENCE OF A PLANT ANTIGEN IN A PLANT"

### BACKGROUND OF THE INVENTION

This invention relates to a method of detecting the presence of a plant antigen such as a virus in or on a plant using an ELISA system, and more specifically to a method for detecting grapevine virus A (GVA) in the tissues of growing and dormant vines in the field.

Plant antigen assays by ELISA techniques are well documented. The assay procedures fall into two categories:

i) direct procedures in which the plant antigen is detected by a specific enzyme-antibody conjugate and ii) indirect procedures in which the plant antigen-specific antibody is used as an intermediate with a second antibody-enzyme conjugate as the final layer. Both procedures are generally based on a double antibody sandwich (DAS) in which the lower layer is the trapping or capture antibody and the upper layer is the detecting antibody. In the case of direct ELISA the detecting antibody is the one conjugated to the enzyme. In the indirect ELISA the detecting antibody is unconjugated.

In all these cases, covalent enzyme-antibody conjugates are used in the detection system.

In the case of GVA, two recent reports exist on the use of ELISA systems (Tanne E. and Givony L. (1985) Phytopath. medit.24 106-109 and Rosciglione B. and Gugerli P. (1986) Revue Suisse Vitic. Hortic. 18 207-217). In both reports, only a single species antiserum was available and it can be concluded that direct DAS ELISA was used. The sensitivity only permitted the detection of GVA in glasshouse grown virus or extracts of field grown vines concentrated 50 fold by ultracentrifugation. No reports exist of ELISA detection of GVA in unconcentrated vine extracts from the field, presumably because existing systems are not sufficiently sensitive. There is thus a need for a method of detecting plant antigens such as viruses, in specific GVA, in plants, which is sufficiently sensitive.

### SUMMARY OF THE INVENTION

According to the invention there is provided a method of detecting the presence of a plant antigen such as a plant virus in or on a plant which includes the steps of:

(a) immobilising a coating antibody to the plant antigen on a surface;

(b) bringing an extract of the plant into contact with the coating antibody to allow the plant antigen which may be present in the plant extract to be bound by the coating antibody;

(c) bringing a detecting antibody to the plant antigen into contact with the bound plant antigen to allow the plant antigen to be bound to the detecting antigen;

(d) bringing a bridging antibody into contact with the detecting antibody to allow the bridging antibody to be bound to the detecting antibody;

(e) bringing an enzyme anti-enzyme antibody complex into contact with the detecting antibody to allow the enzyme anti-enzyme antibody complex to be bound to the bridging antibody;

(f) bringing a sustrate for the enzyme into contact with the bound enzyme anti-enzyme antibody complex; and

(g) detecting any change of the substrate.

According to another aspect of the invention, there is provided a kit for use in a method of detecting a plant antigen in or on a plant, the kit including:

(i) an amount of a coating antibody to the plant antigen;

(ii) an amount of a detecting antibody to the plant antigen;

(iii) an amount of an enzyme anti-enzyme antibody complex;

(iv) an amount of a bridging antibody for the detecting antibody and the enzyme anti-enzyme antibody complex; and

(v) an amount of a substrate for the enzyme.

The kit may optionally include the required buffers and the like.

The enzyme anti-enzyme antibody complex may be a complex derived from any enzyme thta can be used in immunoassays. Examples of such enzymes are alkaline phosphatase, glucose oxidase, B-galactosidase, urease and peroxidase. A preferred enzyme anti-enzyme antibody complex is peroxidase anti-peroxidase (PAP).

### BRIEF DESCRIPTION OF THE DRAWINGS

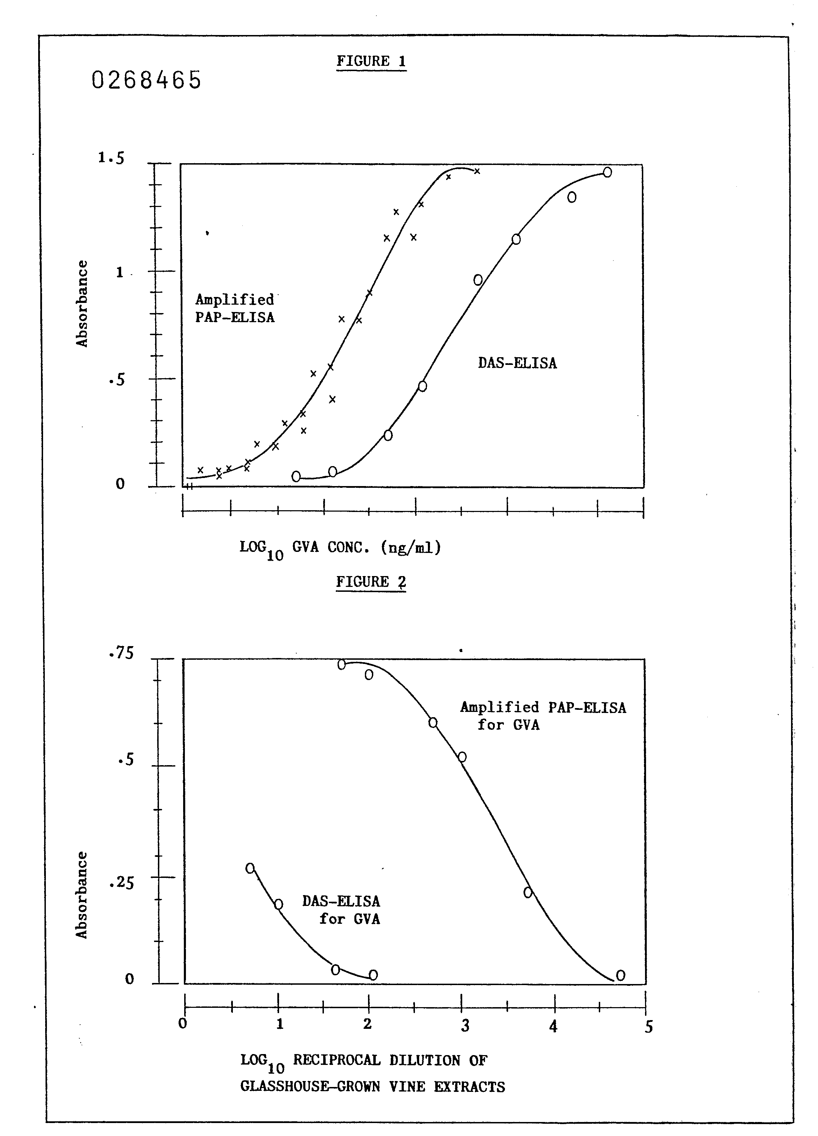

Figures 1 to 4 are graphs illustrating the results obtained utilising the method of the invention and the results obtained utilising a conventional method for the detection of a plant antigen in a plant.

### DESCRIPTION OF PREFERRED EMBODIMENTS

The method of the invention may be used for detecting the presence of any plant antigen in or on a plant. The method is of particular application to the detection of plant viruses in plants and the following description will be based on this application, although it is not limited thereto.

The first step of the method of the invention comprises immobilising a coating antibody to the plant virus on a surface. Generally, the coating antibody will be immobilised on the surface of a suitable test vessel such as a 96 well microtitre plate. The immobilisation may be achieved by coating the coating antibody, suitably diluted in a coating buffer such as a phosphate buffered saline, pH 7,4 for 2 hours at 37°C. Thereafter, the plate is washed, for example with the coating buffer, to remove any unimmobilised coating antibody from the plate. The coating antibody must be specific to the plant virus which is to be detected and may be made in any suitable animal species (hereinafter referred to as species 1) such as sheep, goat, horse, donkey, guinea pig, mouse, rat, chicken or rabbit.

Thereafter, spare or unoccupied sites on the surface of the plate may be blocked with a suitable blocking agent to prevent the detecting antibody from becoming immobilised on the surface of the plate. This may be achieved by applying the blocking agent which may be for example a protein such as casein in a dilution buffer, e.g. 0,5 percent casein in pH 7,6 Tris HCl/saline buffer, to the plate and incubating the plate for 2 hours at 37°C. Thereafter the plate may be washed with a suitable wash buffer such as phosphate buffered saline, pH 7,4 plus 0,1 percent Tween 20.

In the second step of the method of the invention a extract of the plant in a suitable buffer such as the block/dilution buffer (BB) (0,5 percent casein in pH 7,6 Tris HCl/saline buffer) is added into each well of the plate, for example in an amount of 100 μl per well. The plate may then be incubated to ensure binding of the plant virus, if present in the plant extract, to the coating antibody. For example, the plate may be incubated for 16 to 18 hours at 4°C. The incubation may be carried out, for example at 4°C so as to prevent denaturation of the virus.

After the incubation is complete, the plate is washed using a suitable wash buffer.

In the third step of the method of the invention, a detecting antibody to the plant virus is added to the plate. The detecting antibody must again be specific to the virus and may be made in any suitable animal species (species 2) which does not cross-react with the species 1. For example, if the coating antibody is raised in a sheep then the detecting antibody must be raised in an animal other than sheep or goat. The detecting antibody is added to the plate suitably diluted in a buffer solution such as BB, in an amount of for example, 100 μl per well. Incubation is then carried out, for example for 2 hours at 37°C to ensure that binding takes place between the plant virus and the detecting antibody. Once again after the incubation is complete the plate is washed with a suitable wash buffer.

In the fourth step of the method of the invention a bridging antibody is added to the plate. The bridging antibody is designed to bridge between the detecting antibody and the enzyme anti-enzyme antibody complex which is added in the next step. The bridging antibody must be raised in a species (species 3) which does not cross-react with the species 2, and which does not cross-react with the species (species 4) in which the antibody of the enzyme anti-enzyme antibody complex is raised. The antibody of the enzyme anti-enzyme antibody complex is preferably raised in the same species as the detecting antibody, i.e. species 2 and species 4 are the same.

Again, the bridging antibody will generally be added to the plate diluted in a suitable buffer such as BB in a suitable amount such as 100 μl per well. Thereafter, incubation is carried out to ensure binding of the bridging antibody to the detecting antibody, e.g. for 2 hours at 37°C. Thereafter, the plate is washed with a suitable wash buffer.

In the fifth step of the method of the invention, an enzyme anti-enzyme antibody complex is added to the plate. Generally, again the complex will be added suitably diluted in a suitable buffer such as BB, in an amount of for example 100 μl per well. Again,

incubation is carried out, for example for 2 hours at 37°C and thereafter the plate is washed with a suitable wash buffer.

As is stated above, the enzyme anti-enzyme antibody complex may be derived from any enzyme that can be used in immunoassays. The preferred enzyme anti-enzyme antibody complex is peroxidase anti-peroxidase (PAP).

In the sixth step of the method of the invention a substrate for the enzyme is added to the plate. The substrate will generally be a chromogenic compound which undergoes a chemical reaction catalysed by the enzyme which involves a colour change. Alternatively, the substrate may undergo a chemical reaction which involves a change of pH which can be detected by means of a colour indicator. When PAP is used as the enzyme anti-enzyme antibody complex, the substrate may be ABTS (2,2-azinodi-(3-ethylbenzothiazoline sulfone-6) or TMB (3,3′,5,5′-tetramethylbenzidine). Again, the substrate will generally be added in a suitable buffer.

The final step of the method of the invention is to detect any change of the substrate, e.g. ,the colour change, for example with a spectrophotometer.

It is to be noted that the antibodies (coating, detecting and bridging) may be monoclonal or polyclonal antibodies.

To illustrate the effectiveness of the method of the invention a comparison was carried out between the method of the invention (referred to hereinafter as PAP ELISA) and the conventional DAS ELISA routinely used in plant virus detection in the detection of plant viruses. Details of this comparison are given below.

MATERIALS AND METHODS

1. Antisera and complexes used in the PAP ELISA's

Antisera produced in the various species used in this experiment were either obtained commercially, or were prepared as described below.

     a) Rabbit anti-GVA serum. This was prepared using purified virus injected by a standard immunisation protocol with Freund's adjuvant. (GVA = Grapevine Virus A).

     (b) Goat anti-GVA serum. As above.

     (c) Sheep anti-rabbit immunoglobulin serum.

     (d) Rabbit anti-PVY serum. Prepared using purified virus injected by a standard immunisation protocol with Freund's adjuvant. (PVY = Potato Virus Y).

     (e) Sheep anti-PVY serum purchased from Boehringer-Mannheim.

     (f) Rabbit peroxidase anti-peroxidase complex (PAP). Purchased from Pelfreez Biologicals of Arkansas, U.S.A.

2. DAS ELISA Systems (as described by Clark & Adams, (1977) J. Gen. Virol. 34 475-483).

     (a) For GVA. Rabbit anti-GVA serum was processed by ammonium sulphate precipitation, ion exchange chromatography and gel-filtration to yield immunoglobulin (IgG). This IgG was covalently coupled to alkaline phosphatase by a standard glutaraldehyde method. The subsequent conjugate was used as the detection layer in the DAS ELISA with p-nitrophenyl phosphate as the substrate. Absorbance is read at 405 nm.

(b) For PVY. A commercially available anti-PVY kit (Boehringer-Mannheim) was used as per manufacturers instructions. The system also utilises IgG conjugated to alkaline phosphatase and measures absorbance at 405 nm.

## 3. GENERAL PROTOCOL FOR PAP-ELISA SYSTEMS

### A. COMPONENTS

1. Antibodies
   (a) Coating antiserum; species 1 (goat, sheep or chicken)
   (b) Detecting antiserum species 2 (rabbit)
   (c) Bridging antiserum; species 1 antispecies 2 (e.g. sheep anti-rabbit immunoglobulin)
   (d) Enzyme complex; rabbit PAP.

2. Buffers
   (a) Coating buffer; phosphate buffered saline, pH 7,4
   (b) Wash buffer; PBS plus 0,1% Tween 20
   (c) Block/dilution buffer; 0,5% casein in pH 7,6 Tris HCl/saline buffer
   (d) Substrate buffer; 0,1 M citrate buffer pH 5,0 for ABTS or 0,1 M acetate buffer pH 5,8 for TMB.

3. Substrates
   Either of the following:
   ABTS (2,2-azinodi-(3-ethylbenzothiazoline sulfone-6) 0,5 mg/ml in citrate buffer plus $H_2O_2$ to final conc. of 0,015% w/v.
   or
   TMB (3,3′,5,5′-tetramethylbenzidine), 0,1 mg/ml in acetate buffer plus $H_2O_2$ to f. con. of 0,006% w/v.

### B. STEPS

1. Coat microtitre plate (NUNC Immunoplate) with 150 μl of coating antiserum suitably diluted in PBS for 2 hours at 37°C.
2. Wash plates by filling and emptying wells 6 times with PBS alone.
3. Add 200 μl of block/dilution buffer (BB) per well. Incubate 2 hours at 37°C.
4. Wash as in 2 using wash buffer.
5. Dilutions of virus or plant extract in BB, 100 μl per well. Incubate 16-18 hours at 4°C.
6. Wash as in 4.
7. Detecting antiserum, suitably diluted in BB, 100 μl per well, 2 hours at 37°C.
8. Wash as in 4.
9. Bridging antiserum, suitably diluted in BB 100 μl per well, 2 hours at 37°C.
10. Wash as in 4.
11. Rabbit PAP suitably diluted in BB, 100 μl per well 2 hours at 37°C.
12. Wash as in 4.
13. Add substrate, 150 μl per well; for ABTS incubate at room temperature for 30-60 minutes and read adsorbance at 405 nm on plate reader (e.g. Titertek Multiscan). For TMB incubate for 10 minutes add 50 μl 3M $H_2SO_4$ and read at 450 nm.

### 4. Computation

ELISA titrations curves were fitted to the data on an IBM personal computer using "Curve Fitter" programme (P.K. Warme, Interactive Microware Inc. USA). Polynomial interpolation by least squares analysis was used.

## RESULTS

### 1. Purified GVA Detection

Figure 1 shows the detection of purified GVA. From these curves the lowest concentration at which GVA can be detected using the two systems is 8,50 ng/ml by DAS- ELISA and 0,42 ng/ml by PAP-ELISA. These values are based on a positive-negative threshold of absorbance reading 0,1.

### 2. GVA Detection in Vine Tissue Extracts

Petioles or bark from virus grown in a glasshouse or in the field were macerated in liquid nitrogen and extracted with 0,1 M phosphate buffer (pH 7,0) containing 2% PVP and 0,5% casein. The extracts were adjusted to 20% w/v. Figure 2 shows the titration curves for glasshouse-grown material. The sensitivity increase obtained with the PAP ELISA is considerably greater than was observed using the purified virus; threshold values of 1 in 20 for DAS and 1 in 13183 for PAP. This may be due to inhibitory factors present in plant extract, affecting the DAS ELISA more than the PAP ELISA.

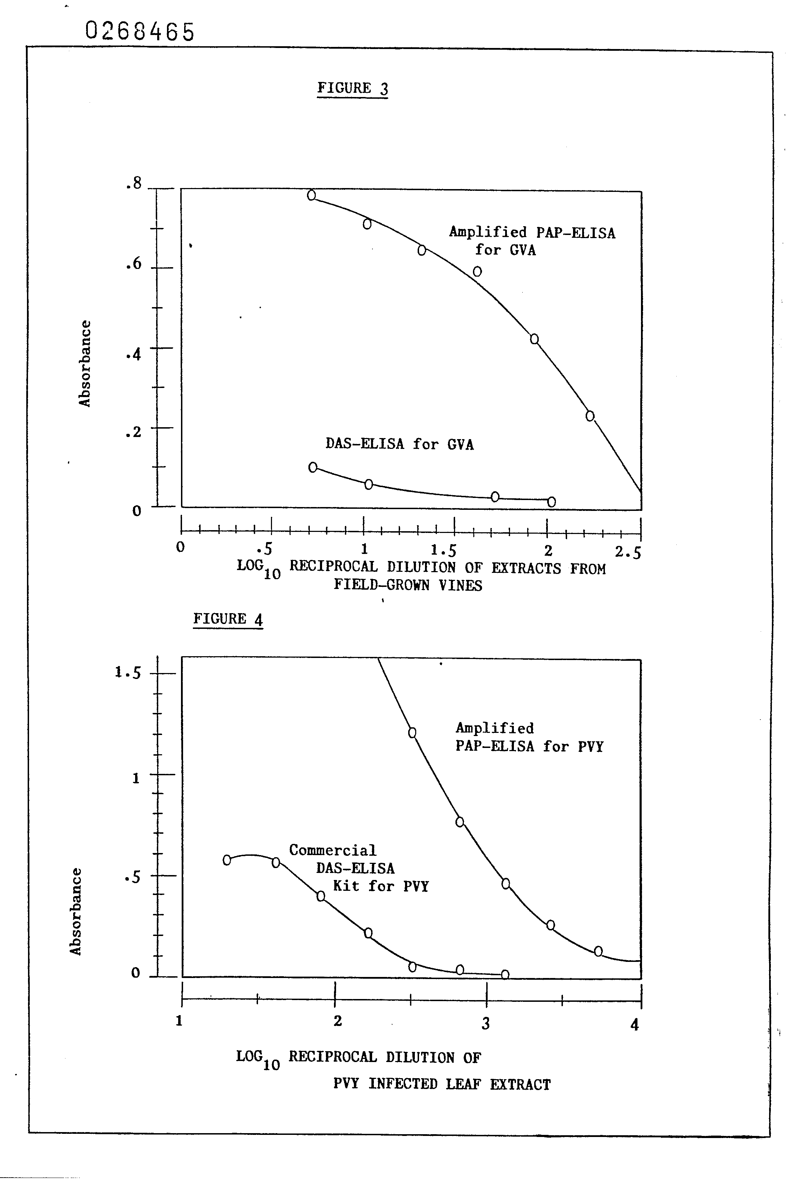

Figure 3 shows the ELISA results of tests on field grown material. In this case the DAS ELISA values do not exceed the 0,1 threshold even at a dilution of 1 in 5. By contrast, the threshold PAP ELISA dilution is 1 in 258.

### 3. PVY Detection in Leaf Extracts

The PVY virus is generally propogated for isolation purposes in a tobacco indicator plant (N. tabacum cv Samson). Extracts of infected tobacco or healthy control tobacco leaves were made at 5% (w/v) in phosphate buffered saline (PBS). Dilutions of leaf extracts were dispensed into microtitre wells coated with sheep anti-PVY (Boehringer-Mannheim, PVY kit) according to manufacturers instructions. Detection was then carried out using either the DAS-ELISA kit system as supplied or the rabbit anti-PVY serum plus amplified PAP-ELISA. Figure 4 shows the two titration curves. The threshold value for DAS is a dilution fo 1 in 244 and for PAP 1 in 7350.

## CONCLUSION

The PAP ELISA has been shown to give considerably increased sensitivity for the detection of two plant viruses, compared with the conventionally used DAS ELISA system. This increase, in the case of GVA is field grown vines, has enabled detection of this virus in natural conditions for the first time by an enzyme-linked immunoassay without the need for concentration of the extract.

The advantages of the method of the present invention (e.g. the PAP-ELISA system) over other systems such as the DAS ELISA system for plant antigen, e.g. plant virus detections are many. Firstly, the method of the invention provides increased sensitivity. This is illustrated by the results given above. Secondly, the method of the invention provides for reproducibility. Replacement of enzyme antibody conjugates with enzyme anti-enzyme antibody complexes gives a standard, more stable reagent with definable characteristics and this provides a highly reproducible detection system. Thirdly, the method of the invention provides for the simplification of reagents. Since no enzyme-antibody conjugates are used, the trapping and detecting antibodies are used as simple diluted sera; no isolation of immunoglobulin is required. Finally, the method of the invention provides for universality of amplification detection system. Any system based, e.g. on rabbit sera as the detecting antibody will use the same bridging agent, enzyme anti-enzyme antibody complex and substrate. These components can thus be used for an extensive range of ELISA's.

The method of the invention has been illustrated for the detection of GVA and PVY. The method may be used for the detection of other plant viruses such as Arabis Mosaic virus on hops, and other plant antigens.

The second aspect of the present invention is a kit for carrying out the method of the invention. The kit will generally contain all the reagents required to carry out the method of the invention, specifically, the essential reagents, viz, the coating antibody, the detecting antibody, the bridging antibody, the enzyme anti-enzyme antibody complex and the substrate for the enzyme. The kit may also contain the various buffers needed. In addition, the kit may contain control samples, e.g. a sample which contains a known quantity of a specific plant antigen.

## Claims

1. A method of detecting the presence of a plant antigen in or on a plant characterized in that it includes the steps of:
(a) immobilising a coating antibody to the plant antigen on a surface:
(b) bringing an extract of the plant into contact with the coating antibody to allow the plant antigen which may be present in the plant extract to be bound by the coating antibody;
(c) bringing a detecting antibody to the plant antigen into contact with the bound plant antigen to allow the plant antigen to be bound to the detecting antibody;
(d) bringing a bridging antibody into contact with the detecting antibody to allow the bridging antibody to be bound to the detecting antibody;
(e) bringing an enzyme anti-enzyme antibody complex into contact with the detecting antibody to allow the enzyme anti-enzyme antibody complex to be bound to the bridging antibody;
(f) bringing a substrate for the enzyme into contact with the bound enzyme anti-enzyme antibody complex; and
(g) detecting any change of the substrate.

2. A method according to claim 1 characterized in that the plant antigen is a plant virus.

3. A method according to claim 2 characterized in that the plant is a grapevine and the virus is Grapevine Virus A.

4. A method according to claim 2 characterized in that the plant is a potato and the virus is Potato Virus Y.

5. A method according to any one of claims 1 to 4 characterized in that the coating antibody is a coating antibody raised in a first animal species and the detecting antibody is a detecting antibody raised in a second animal species which does not cross-react with the first animal species.

6. A method according to claim 5 characterized in that the bridging antibody is a bridging antibody raised in a third animal species and the antibody of the enzyme anti-enzyme antibody complex is raised in a fourth animal species, such that the third animal species does not cross-react with the second animal species of the fourth animal species.

7. A method according to claim 6 characterized in that the fourth species is the same as the second species.

8. A method according to claims 1 to 7 characterized in that the enzyme anti-enzyme antibody complex is peroxidase anti-peroxidase.

9. A method according to claim 8 characterized in that the substrate is selected from 2,2-azinodi-(3-ethylbenzothiazoline sulfone-6) and 3,3',5,5'-tetramethylbenzidine.

10. A kit for use in a method of detecting a plant antigen in or on a plant is characterized in that it includes:
(i) an amount of a coating antibody to the plant antigen;
(ii) an amount of a detecting antibody to the plant antigen;
(iii) an amount of an enzyme anti-enzyme antibody complex;
(iv) an amount of a bridging antibody for the detecting antibody and the enzyme anti-enzyme antibody complex; and
(v) an amount of a substrate for the enzyme.

**FIGURE 1**

LOG$_{10}$ GVA CONC. (ng/ml)

**FIGURE 2**

LOG$_{10}$ RECIPROCAL DILUTION OF
GLASSHOUSE-GROWN VINE EXTRACTS

0268465

FIGURE 3

Amplified PAP-ELISA for GVA

DAS-ELISA for GVA

Absorbance

$LOG_{10}$ RECIPROCAL DILUTION OF EXTRACTS FROM FIELD-GROWN VINES

FIGURE 4

Amplified PAP-ELISA for PVY

Commercial DAS-ELISA Kit for PVY

Absorbance

$LOG_{10}$ RECIPROCAL DILUTION OF PVY INFECTED LEAF EXTRACT